(19) Europäisches Patentamt
European Patent Office
Office européen des brevets

(11) Veröffentlichungsnummer: **0 116 621**
**B1**

(12) **EUROPÄISCHE PATENTSCHRIFT**

(45) Veröffentlichungstag der Patentschrift: **13.05.87**

(51) Int. Cl.⁴: **A 61 K 7/00**, C 07 C 69/24

(21) Anmeldenummer: **83902807.3**

(22) Anmeldetag: **26.08.83**

(86) Internationale Anmeldenummer: **PCT/EP 83/00225**

(87) Internationale Veröffentlichungsnummer: **WO 84/00884 (15.03.84** Gazette **84/7)**

(54) **VERWENDUNG VON ESTERN VERZWEIGTKETTIGER CARBONSÄUREN MIT VERZWEIGTKETTIGEN ALKOHOLEN ALS BESTANDTEIL KOSMETISCHER MITTEL.**

(30) Priorität: **26.08.82 DE 3231705**
**26.08.82 DE 3231706**

(43) Veröffentlichungstag der Anmeldung: **29.08.84 Patentblatt 84/35**

(45) Bekanntmachung des Hinweises auf die Patenterteilung: **13.05.87 Patentblatt 87/20**

(84) Benannte Vertragsstaaten: **CH DE FR GB LI NL**

(56) Entgegenhaltungen:
**FR - A - 1 486 689**
**FR - A - 2 486 800**
**US - A - 3 188 330**

**Chemical Abstracts, Band 90, Nr. 14, 2. April 1979, Columbus, Ohio (US), siehe Seite 374, Spalte 1, Zusammenfassung 109806b,JP-A-78 127 839 (KANEBO), 8.Nov.1978**
**Tenth Collective Index, Seite 25334 "Hexanoic acid, 2-ethyl, isooctadecyl ester"; Seite 28896 "Isononanoic acid, isodecylester u. isononyl ester"**
**Estrin et. al. (eds.): "CTFA Cosmetic Ingredient Dictionary", 3.Auflage1982, CTFA, Washington, US, siehe Seite 136, isodecyl isononanoate; Seite 137,**

(73) Patentinhaber: **Dragoco Gerberding & Co. GmbH, Dragocostrasse 1, D-3450 Holzminden (DE)**

(72) Erfinder: **BRUNKE, Ernst-Joachim, Holbeinstrasse 6, D-3450 Holzminden (DE)**
Erfinder: **ROJAHN, Willi, Wiesenweg 35, D-3450 Holzminden (DE)**

(74) Vertreter: **Patentanwälte Müller-Boré, Deufel, Schön, Hertel, Lewald, Otto, Postfach 26 02 47 Isartorplatz 6, D-8000 München 26 (DE)**

(56) Entgegenhaltungen: (Fortsetzung)
**isononyl isononanoate; Seite 142, isotridecyl isononanoate**

**Die Akte enthält technische Angaben, die nach dem Eingang der Anmeldung eingereicht wurden und die nicht in dieser Patentschrift enthalten sind.**

**EP 0 116 621 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die für die Pflege der menschlichen Haut verwendeten kosmetischen Produkte bestehen aus Grundmassen, die speziell auf die jeweilige Verwendung abgestimmt sind. In diese Grundmassen können verschiedenartige Wirkstoffe und Parfümöle eingearbeitet werden. Die kosmetischen Grundmassen sollen aber nicht nur ein Träger für Wirkstoffe sein, sondern selbst auch schon möglichst positive Effekte auf der Haut ausüben, z.B. die Hautoberfläche gegen äussere Schädigungen schützen und ein ausgewogenes Verhältnis von Feuchtigkeits- und Fettgehalt der Haut bewirken.

Die in der Kosmetik üblicherweise verwendeten Cremes und Lotionen sind Emulsionen aus Kohlenwasserstoffen, Fetten, natürlichen oder synthetischen Wachsen, Emulgatoren und Wasser. Konsistenz der Emulsion und Hautwirkung hängen von der Art und der Konzentration der Bestandteile ab. Es hat sich gezeigt, dass für ein kosmetisches Produkt eine möglichst hohe Wasserdampfdurchlässigkeit auf der Haut wünschenswert ist, da somit die normale Hautfunktion nicht beeinträchtigt wird. Die in grossen Mengen verwendeten natürlichen Fette sind oft Triglyceride geradkettiger Fettsäuren (z.B. Stearin- und Palmitinsäure). Derartige höhermolekulare Triglyceride behindern auf der Haut den Durchtritt von Wasserdampf; die verzweigtkettigen Analoga haben hierbei deutlich günstigere Eigenschaften (G. Weitzel et al., Hoppe-Seyler's Zeitschrift. Physiol. Chemie, 301, 26 (1955)).

Dementsprechend sind von der kosmetischen Industrie verschiedene verzweigtkettige Verbindungen (Kohlenwasserstoffe, Carbonsäuren, Alkohole und Ester) entwickelt worden, die auch zur Erhöhung der Spreitfähigkeit des Kosmetikums verwendet werden (Übersicht in: M.G. de Navarre, American Perfumer and Cosmetics, 79 (1936)). Die Wirkung verzweigtkettiger Ester in Hautpflegeprodukten wurde als besonders günstig ermittelt (J.S. Jellinek, Cosmetics and Toiletries 95, 69 (1978)).

Als eines der zur Zeit wirksamsten Produkte dieser Gruppe hat sich das «PCL-liquid» erwiesen (A. Berg, DRAGOCO-Report, 159 (1976)). Hierbei handelt es sich im wesentlichen um die Verbindungen 1, 2, das Gemisch der Ester von 2-Ethylhexansäure und den geradkettigen $C_{16}/C_{18}$-n-Alkoholen.

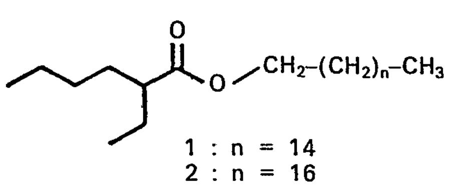

1 : n = 14
2 : n = 16

Aus Chemical Abstracts 90 (1979) 109806 b, FR-A 2 486 800 und Estrin et al., CTFA Cosmetic Ingredient Dictionary 1982, S. 136, 137 und 142, sind Ammoniumsalze von Fettsäureestern, 3,3,5-Trimethylhexansäureisodecylester, Isotridecylisononanoat sowie Isononylisononanoat als Salbengrundlage bekannt, doch wird in diesen Druckschriften nichts über die eine gleichmässige Verteilung von Wirkstoffen auf der Haut ermöglichende Spreitfähigkeit dieser Verbindungen gesagt.

Es ist die Aufgabe der vorliegenden Erfindung, durch Verwendung neuer oder nur als chemische Individuen bekannter Substanzen, über deren Eignung für kosmetische Zwecke jedoch bisher noch nichts bekannt war, die Eigenschaften von Kosmetika zu verbessern.

Insbesondere soll durch die Verwendung von noch nicht für derartige Zwecke eingesetzten Substanzen die Wasserdampfdurchlässigkeit von Kosmetika auf der Haut verbessert werden und die gleichmässigere Verteilung von Wirkstoffen auf der Haut möglich sein.

Es ist ferner Aufgabe der vorliegenden Erfindung, neue kosmetische Mittel anzugeben, die aufgrund eines Gehalts an bisher in Kosmetika nicht verwendeten Substanzen verbesserte Eigenschaften aufweisen.

Diese Aufgaben werden durch die Verwendung der in den Patentansprüchen bezeichneten Substanzen bzw. Mittel mit einem Gehalt an diesen Substanzen gelöst.

Die durch besondere Unteransprüche hervorgehobenen Teilgruppen der in den Hauptansprüchen bezeichneten Gruppen von Substanzen umfassen besonders geeignete Substanzen.

Die vorliegende Erfindung betrifft somit die Verwendung von Substanzen der allgemeinen Formel A bzw. kosmetische Mittel mit einem Gehalt einer wirksamen Menge einer solchen Verbindung A.

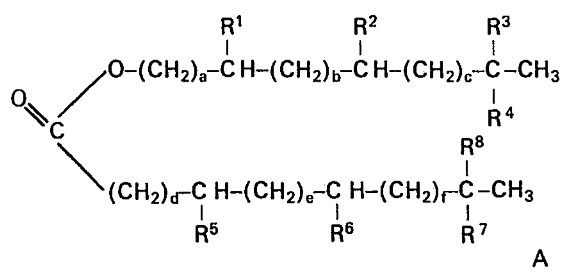

A

$R^1, R^2, R^3, R^4, R^5, R^6, R^7, R^8$ = H, Me, Et
a + b + c = 2–13
d + e + f = 1–2

Es wurde gefunden, dass die erfindungsgemässen Substanzen der allgemeinen Formel A eine ausgesprochen hohe Spreitfähigkeit besitzen und auch das Estergemisch 1, 2 darin übertreffen. Dadurch wird die Wasserdampfdurchlässigkeit von Kosmetika, die die Verbindungen A enthalten, auf der Haut deutlich verbessert. Ausserdem verleihen die Verbindungen A verschiedenen kosmetischen Mischungen die Eigenschaft, sich sehr gut auf der Haut zu verteilen. Eine gleichmässige Verteilung auf der Haut ist günstig für die Aufbringung bestimmter Wirkstoffe (z.B. Lichtschutzfiltersubstanzen) durch ein Kosmetikum.

Die hohe Spreitfähigkeit der Verbindungen der allgemeinen Formel A kann als Folge der mehrfachen Substitution des Moleküls angesehen werden. Sowohl der Alkohol als auch der Säureteil sind verzweigtkettig. Die Substanzen der Formel A sind bei Raumtemperatur Flüssigkeiten mittlerer Viskosität und können gut verarbeitet werden. Aufgrund der mehrfachen Verzweigung und fehlender Unsättigungen sind die Verbindungen A nur schwer autoxidierbar und weisen somit praktisch keine Ranzidität auf. Die Ester der Formel A können daher vorteilhaft einzeln oder im Gemisch als Bestandteil kosmetischer Produkte verwendet werden. Hautöle (z.B. Sonnenschutzöle) können die Ester der Formel A zu 90% enthalten. Aufgrund der hohen Spreitfähigkeit der Verbindungen A können auch grössere Mengen von Paraffinöl im Gemisch verwendet werden, als dies sonst wünschenswert ist (Verkleben der Hautporen). Die Dosierung der Ester A in kosmetischen Produkten beträgt vorzugsweise 2–30%.

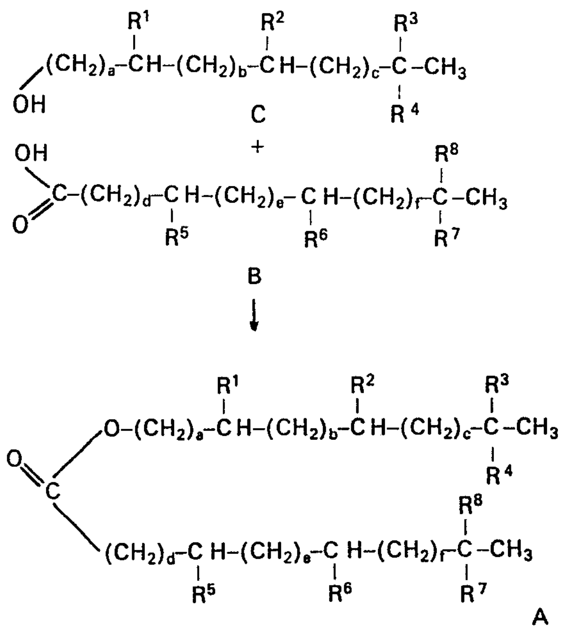

C

B

A

$R^1, R^2, R^3, R^4, R^5, R^6, R^7, R^8$ = H, Me, Et
a + b + c = 2–13
d + e + f = 1–2

Bei den erfindungsgemässen Estern der allgemeinen Formel A können $R^1$, $R^2$, $R^3$ und $R^4$ bzw. $R^5$, $R^6$, $R^7$ und $R^8$ nicht gleichzeitig Wasserstoffatome bedeuten, was direkt aus der Angabe «verzweigtkettig» sowohl für den Alkohol als auch die Säure folgt. Die Symbole a, b, c, d, e, f können individuell jeden beliebigen ganzzahligen Wert von 0 bis 13 annehmen, solange die Randbedingungen a + b + c = 2–13 bzw. d + e + f = 1–2 erfüllt sind.

Die Darstellung der Ester A erfolgt durch übliche Veresterung der entsprechenden verzweigtkettigen Alkohole und Säuren, bzw. durch Umsetzen der verzweigtkettigen Alkohole mit den Säurechloriden.

Als Carbonsäuren können verschiedene industriell erzeugte einfach oder mehrfach verzweigte Octansäuren B (z.B. 2-Ethylhexansäure oder «Isooctansäure») und als Alkohole industriell erzeugte, einfach oder mehrfach verzweigte Alkohole C (z.B. 2-Ethylhexanol, Isooctanol-Isomere, Isononanol, Isodecanol-Isomere, Isotridecanol-Isomere, Isooctadecanol-Isomere) verwendet werden.

2-Ethylhexansäure (3) und 2-Ethylhexanol werden industriell in grossen Mengen erzeugt und in Kunststoffen verarbeitet. Von den höheren Estern der Formel A der Ethylhexansäure ist der 2-Ethylhexansäure-2-ethylhexylester eine bekannte

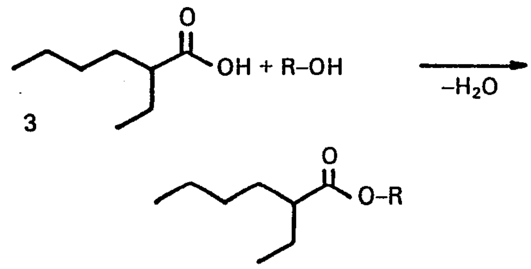

3

$R^1, R^2, R^3, R^4, R^5, R^6, R^7, R^8$ = H, Me, Et
a + b + c = 2–13
d + e + f = 1–2

z.B. α-Ethylhexyl-
Isooctyl-(Isomere)
3.3.5-Trimethylhexyl-(Isononyl-)
Isodecyl-(Isomere)
Isotridecyl-(Isomere)

Substanz. So wurde er als Nebenprodukt bei der Synthese von 2-Ethylhexanol aus Butyraldehyd erhalten (K. Nagaoka, T. Adachi und S. Kudo, Kogyo Kagaku Zasshi 66, 231, 1822 (1963) ). Die Neth. Appl. 6 510 136 (Feb. 7, 1966) beschreibt die Eignung dieses Esters als Mittel zur technischen Schaumverhinderung. Die Darstellung des genannten Esters kann durch übliche säurekatalysierte Veresterung von 2-Ethylhexansäure (3) und 2-Ethylhexanol, bzw. durch Umsetzen von 2-Ethylhexanol mit dem entsprechenden Säurechlorid erfolgen. Eine weitere Produktionsmethode (Verwendung von Molybdänsulfid/Aktivkohle) ist in dem U.S. Patent 3 329 826 (Juli 4, 1967) beschrieben worden. Der 2-Ethylhexansäure-2-ethylhexylester kann auch durch partielle edelmetallkatalysierte Oxidation des 2-Ethylhexanols dargestellt werden (Fr. Pat. 1 563 259 vom 11. April 1969). In der Franz. Anm. 2 282 467 (19. März 1976) wird die Verwendung des genannten Esters als Dielektrikum für Transformatoren beansprucht. Weitere Methoden für die Darstellung des Esters sind beschrieben von A.N. Shampolova, V.I. Lyubomilov, R.N. Sivakova und A.Kh. Bulai, Zh. Org. Khim. 1979, 2496 und W. Tamura, Y. Fukenoka, Y. Suzuki und S. Yamamatsu, Jpn. Kokai Tokkyo Koho 80 22 640 (18. Feb. 1980).

Über die Eignung des 2-Ethylhexansäure-2-ethylhexylesters oder der übrigen höheren verzweigtkettigen Ester der 2-Ethylhexansäure als Kosmetik-Bestandteil war nichts bekannt. Es ist daher neuartig, dass sich diese Ester als flüssige

Wachse vorteilhaft zum Aufbau kosmetischer Grundmassen verwenden lassen.

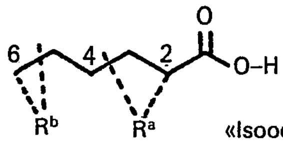

«Isooctansäure»

$R^a$:$CH_3$ alternativ an C-2, C-3 oder C-4
$R^b$:$CH_3$ alternativ an C-5, oder C-6
bzw. C-6 und C-6

R–OH + Isooctansäure → Isooctansäure-Ester
4a–h

R = Ethylhexyl-
Isooctyl-(Isomere)
Isononyl-(Isomere)
Isodecyl-(Isomere)
Isotridecyl-(Isomere)

Die «Isooctansäure» (4) wird industriell durch Oxidation von «Isooctylaldehyd» hergestellt, der wiederum durch Oxosynthese aus einem Heptengemisch (erhalten durch Codimerisation von Propylen und Butylen) entsteht (s.a.: J. Falbe, «Synthesen mit Kohlenmonoxid», 1. Kap. «Hydroformylierungen» (Roelen-Reaktion), Springer-Verlag, Berlin, Heidelberg, New York 1967; H.E. Kyle, «Oxo Process», in: Kirk-Othmer, Bd. 14, 2. Aufl., S. 373–390, J. Wiley & Sons, New York 1967). Dementsprechend liegt die «Isooctansäure» als Gemisch von Konstitutionsisomeren vor. Die bei industrieller Herstellung üblichen Schwankungen der relativen Konzentration der Isomerenzusammensetzung hat keine merkliche Auswirkung auf die vorteilhaften kosmetischen Eigenschaften des Isooctansäure-2-ethylhexylester-Gemischs. Aufgrund des Synthesewegs besteht «Isooctansäure» aus den $C_8$-Carbonsäuren 4a–h. Es sind ferner Methylgruppenwanderungen möglich, die aber nur eine untergeordnete Rolle spielen. Für die massenspektrometrischen Untersuchungen

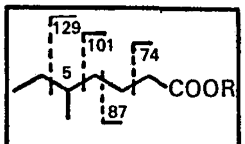

4a:R=H
5a:R=Me
6a:R= 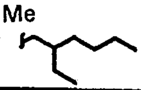

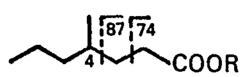

4b:R=H
5b:R=Me
6b:R= 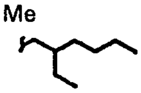

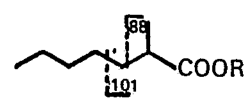

4c:R=H
5c:R=Me
6c:R= 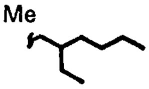

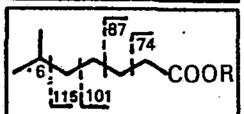

4d:R=H
5d:R=Me
6d:R= 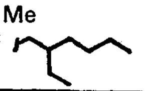

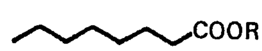

4e:R=H
5e:R=Me
6e:R= 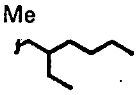

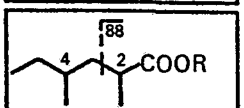

4f:R=H
5f:R=Me
6f:R= 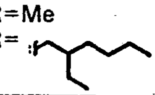

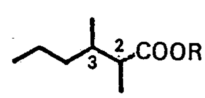

4g:R=H
5g:R=Me
6g:R= 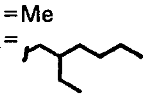

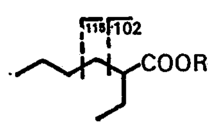

4h:R=H
5h:R=Me
6h:R= 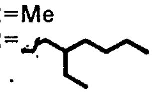

der isomeren Isooctansäuren wurde das Gemisch der Methylester 5a–h dargestellt. Aufgrund der oben angegebenen massenspekrometrischen Fragmentierung wurde die in Abb. 1 gegebene Zuordnung getroffen. Diese Isomerenverteilung wiederholt sich bei den verzweigtkettigen Estern der Formel A, wie anhand der 2-Ethylhexyl-Ester 6a–h exemplarisch aufgezeigt wurde (Gaschromatogramm Abb. 2, Massenspektren der Hauptisomeren Abb. 3).

Die relative Zusammensetzung der isomeren verzweigtkettigen Isooctansäure-Ester der Formel A kann den für grosstechnische Chemieverfahren üblichen Schwankungen unterliegen, ohne dass die Verwendung der Ester in kosmetischen Mitteln beeinträchtigt wird.

Die anschliessenden Beispiele erläutern die Erfindung.

Beispiel 1

Herstellung von 2-Ethylhexansäure-3,5,5-trimethylhexylester

Eine Lösung von 144 g (1 mol) 2-Ethylhexansäure und 144 g (1 mol) 3,5,5-Trimethylhexanol

(Isononylalkohol) in 300 ml Toluol wird mit 2 g konzentrierter Schwefelsäure versetzt und unter Wasserabscheidung zum Sieden erhitzt, bis die equimolare Menge Wasser abgeschieden ist (ca. 2 Stdn.). Nach Abkühlen wurde mit Wasser und anschliessend mit 10%iger $NaHCO_3$-Lösung gewaschen. Das Lösungsmittel wurde unter vermindertem Druck abdestilliert. Das verbleibende Rohprodukt ergab nach Destillation über eine 20 cm-Vigreux-Kolonne 220 g (85%) 2-Ethylhexansäureester als farbloses Öl mittlerer Viskosität; Kp (1 mm) = 118–120 °C.

$d_{4^\circ}^{20^\circ}$ : 0.8555 $n_{D}^{20^\circ}$ : 1.4346

Beispiel 2
Dichte und Brechung einiger 2-Ethylhexansäure-Ester
(Darstellung analog Beispiel 1)
2-Ethylhexansäure-isodecylester (aus Isodecanol-Isomerengemisch der Fa. Hoechst)

$d_{4^\circ}^{20^\circ}$ : 0.8603 $n_{D}^{20^\circ}$ : 1.4382

2-Ethylhexansäure-isotridecylester (aus Tridecanol-Isomerengemisch der Fa. Hoechst)

$d_{4^\circ}^{20^\circ}$ : 0.8629 $n_{D}^{20^\circ}$ : 1.444

2-Ethylhexansäure-isooctadecylester

$d_{4^\circ}^{20^\circ}$ : 0.8569 $n_{D}^{20^\circ}$ : 1.4473

Beispiel 3
Herstellung von «Isooctansäure»-methylester

Eine Lösung von 32 g (1 mol) Methanol und 144 g (1 mol) «Isooctansäure» (Isomerengemisch der Fa. Ruhrchemie A.G., Oberhausen) in 200 ml Toluol wird mit 2 g konzentrierter Schwefelsäure versetzt und unter Wasserabscheidung zum Sieden erhitzt, bis die equimolare Menge Wasser abgeschieden ist (ca. 2 Stdn.). Nach Abkühlen wurde mit Wasser und anschliessend mit 10%iger Natriumhydrogencarbonat-Lsg. gewaschen. Das Lösungsmittel wurde unter vermindertem Druck abdestilliert. Das verbleibende Rohprodukt ergab nach Destillation über eine 15 cm-Vigreux-Kolonne 150 g (95% Isooctansäuremethylester als leichtbewegliche Flüssigkeit mit fruchtigem Geruch.
$Kp_1$ = 58–63 °C

$d_{4^\circ}^{20^\circ}$ : 0.8895 $n_{D}^{20^\circ}$ : 1.4175

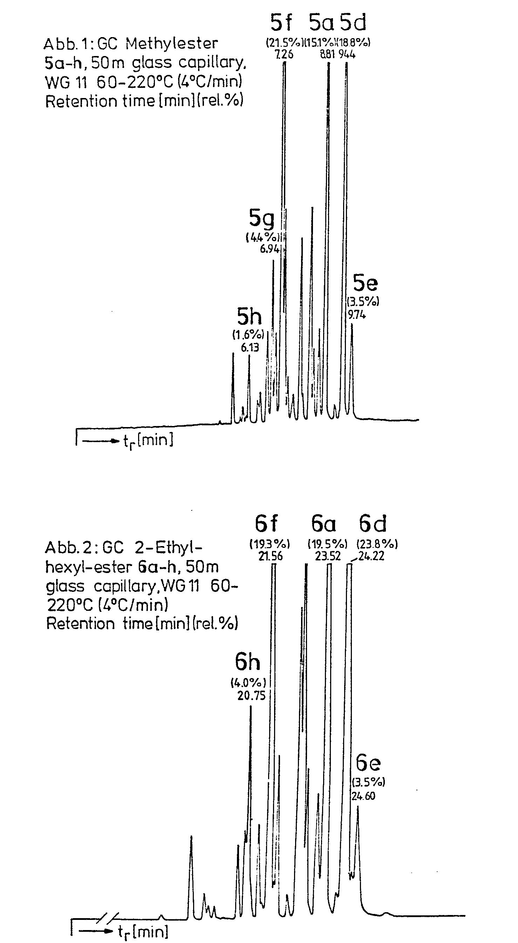

Abb. 1: Gaschromatogramm.

Beispiel 4
Herstellung von Isooctansäure-2-ethylhexylester

Eine Lösung von 130 g (1 mol) 2-Ethylhexanol und 144 g (1 mol) «Isooctansäure» (Isomerengemisch der Fa. Ruhrchemie A.G. Oberhausen) in 300 ml Toluol wird mit 2 g konzentrierter Schwefelsäure versetzt und unter Wasserabscheidung zum Sieden erhitzt, bis die equimolare Menge Wasser abgeschieden ist (ca. 2 Stdn.). Nach Abkühlen wurde mit Wasser und anschliessend mit 10%iger Natriumhydrogencarbonat-Lsg. gewaschen. Das verbleibende Rohprodukt ergab nach Destillation über eine 15 cm.Vigreux-Kolonne 230 g (90%) Isooctansäure-2-ethylhexylester als farbloses Öl mittlerer Viskosität.
$Kp_1$ = 110–117 °C

$d_{D}^{20^\circ}$ : 0.8645 $n_{D}^{20^\circ}$ : 1.4367

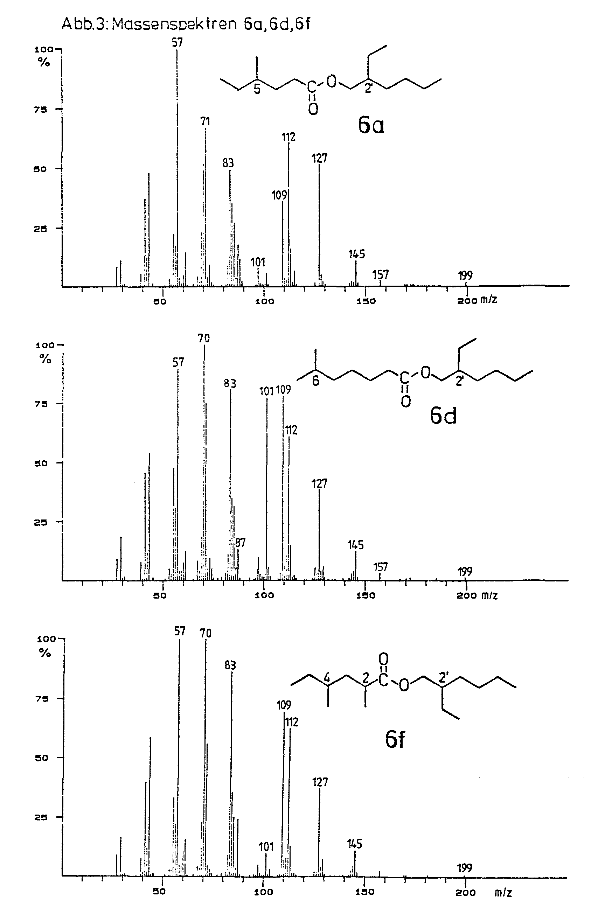

Das Produkt ist ein Isomerengemisch; Abb. 2: Gaschromatogramm. Massenspektren (GC/MS-Kopplung) der Hauptisomeren 6a, 6d und 6f: Abb. 3.

Beispiel 5
Dichte und Brechung einiger Isooctansäure-Ester
Isooctansäure-3,5,5-trimethylhexylester
Darstellung analog Beispiel 9; farblose Flüssigkeit

$d_{4^\circ}^{20^\circ}$ : 0.8603 $n_{D}^{20^\circ}$ : 1.4369

Isooctansäure-isodecylester
Darstellung analog Beispiel 9; farblose Flüssigkeit

$D_{4^\circ}^{20^\circ}$ : 0.8651 $n_{D}^{20^\circ}$ : 1.4409

Isooctansäure-isotridecylester
Darstellung analog Beispiel 9; farblose Flüssigkeit

$d_{4^\circ}^{20^\circ}$ : 0.8667 $n_{D}^{20^\circ}$ : 1.4460

Isooctansäure-isooctadecylester
Darstellung analog Beispiel 9; farblose Flüssigkeit

$d_{4^\circ}^{20^\circ}$ : 0.8559 $n_{D}^{20^\circ}$ : 1.4490

Beispiel 6
Spreitfähigkeits-Bestimmung

Auf ein kreisrundes Filterpapier (∅ = 110 mm, Gewicht 0,81 g; Fa. Schleicher & Schüll, Typ 597) wurden jeweils 0,1 ml Isooctansäure-2-ethylhexylester (6a–h) und des Gemisches 1, 2 mit einer Pipette punktförmig in der Mitte aufgetragen. Die Substanzen verteilten sich durch Kapillarkräfte radial. Nach 20 min Aufbewahren (horizontal) bei Raumtemperatur, wobei das Papier lediglich am Rand auflag, wurde die durch Aufsaugen der Substanz(en) durchsichtig gewordene, fast kreisrunde Fläche planimetrisch bestimmt.

| | Spreitfähigkeit $cm^2$ |
|---|---|
| Paraffinöl 5°E | 19,8 |
| PCL-liquid (1, 2) | 25,1 |
| 2-Ethylhexansäure-2'-ethylhexylester | 36,7 |
| 2-Ethylhexansäure-3,5,5-trimethylhexylester | 34,8 |
| 2-Ethylhexansäure-isodecylester | 27,3 |
| 2-Ethylhexansäure-isotridecylester | 24,2 |
| 2-Ethylhexansäure-isooctadecylester | 19,9 |
| Isooctansäure-2-ethylhexylester | 38,0 |
| Isooctansäure-3,5,5-trimethylhexylester | 26,5 |

| | Spreitfähigkeit $cm^2$ |
|---|---|
| Isooctansäure-isodecylester | 30,2 |
| Isooctansäure-isotridecylester | 28,7 |
| Isooctansäure-isooctadecylester | 20,2 |

Die Spreitfähigkeit der erfindungsgemässen verzweigtkettigen Isooctansäure-Ester erreicht oder übertrifft die des Paraffinöls oder des Marktprodukts 1, 2.

Beispiel 7
Sonnenschutzöl

| | |
|---|---|
| Paraffinöl 5°E | 50 |
| Isopropylmyristat | 25 |
| Mehrfach verzweigter Ester A | 22 |
| p-Methoxy-zimtsäure-octylester | 2 |
| Parfümöl | 1 |
| | 100 |

Das so erhaltene Öl kann jeweils der Haut direkt oder als Aerosol-Spray aufgetragen werden.

Beispiel 8
Tagescreme

| | |
|---|---|
| Glycerinmonostearat/Na-stearat | 11,2 |
| Isopropylmyristat | 3,0 |
| Mehrfach verzweigter Ester A | 2,0 |
| p-Hydroxybenzoesäureethylester | 0,3 |
| Sorbit F | 3,0 |
| Wasser | 80,0 |
| Parfümöl | 0,5 |
| | 100,0 |

Durch Emulgieren bei 60–70 °C wurde eine weiche, «softige» Hautcreme mit guter Stabilität und gutem Einziehvermögen erhalten.

Beispiel 9
Salbengrundlage

| | |
|---|---|
| Paraffinum sol | 4,4 |
| Lanolin anh. | 4,4 |
| Sorbitan-sesquioleat | 2,6 |
| Palmitinsäure-cetylester | 2,2 |
| Lanolinalkohol | 1,8 |
| Ölsäuredecylester | 5,7 |
| Stearinsäure | 0,9 |
| Butylhydroxytoluol | 0,1 |
| p-Hydroxybenzoesäureethylester | 0,3 |
| Mehrfach verzweigter Ester A | 6,0 |
| Magnesiumsulfat | 0,5 |
| 1,2-Propandiol | 3,0 |
| Wasser | 67,6 |
| Parfümöl | 0,5 |
| | 100,0 |

Die durch Emulgieren bei 60–70 °C erhaltene Salbengrundlage besitzt ein sehr gutes Aufziehvermögen.

**Patentansprüche**

1. Verwendung von Estern der allgemeinen Formel A, gebildet aus verzweigtkettigen Octansäuren und verzweigtkettigen Alkoholen, als Mittel zur gleichmässigeren Verteilung von Wirkstoffen auf der Haut in kosmetischen Mitteln

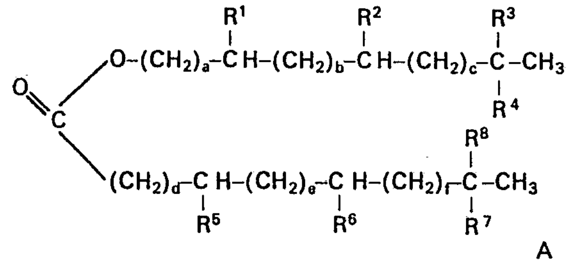

A

$R^1, R^2, R^3, R^4, R^5, R^6, R^7, R^8$ = H, Me, Et
a + b + c = 2–13
d + e + f = 1–2

2. Kosmetische Mittel, gekennzeichnet durch einen Gehalt einer wirksamen Menge von Estern der allgemeinen Formel A, gebildet aus verzweigtkettigen Octansäuren und verzweigtkettigen Alkoholen als Mittel zur gleichmässigeren Verteilung von Wirkstoffen auf der Haut.

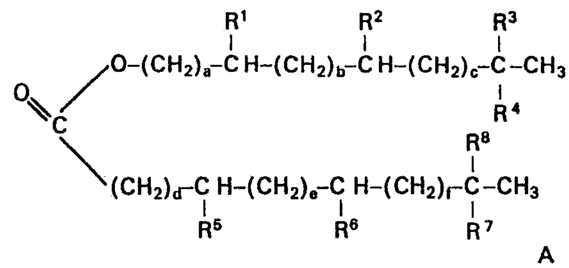

A

$R^1, R^2, R^3, R^4, R^5, R^6, R^7, R^8$ = H, Me, Et
a + b + c = 2–13
d + e + f = 1–2

3. Kosmetische Mittel nach Anspruch 2, dadurch gekennzeichnet, dass die Ester der allgemeinen Formel A verzweigtkettige Isooctansäureester sind, die sich von technischer Isooctansäure der allgemeinen Formel

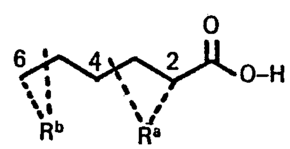

in der $R^a$ eine Methylgruppe am Kohlenstoffatom 2, 3 oder 4 ist, und $R^b$ eine Methylgruppe am Kohlenstoffatom 5 oder 6 ist, in Form eines Isomerengemisches aus einfach oder zweifach methylverzweigten $C_8$-Säuren, sowie von aliphatischen Alkoholen mit 8 bis 18 Kohlenstoffatomen und 1 bis 3 Methyl- oder Ethylseitenketten ableiten.

4. Kosmetische Mittel nach Anspruch 2, dadurch gekennzeichnet, dass die Ester der allgemeinen Formel A verzweigtkettige 2-Ethylhexansäureester sind, die aus 2-Ethylhexansäure und einem aliphatischen Alkohol mit 8 bis 18 Kohlenstoffatomen und 1 bis 3 Methyl- oder Ethylseitenketten gebildet sind.

5. Kosmetische Mittel nach Anspruch 2, dadurch gekennzeichnet, dass sie die Ester der allgemeinen Formel A in einer Menge von 1 bis 90 Gew.-% enthalten.

6. Kosmetische Mittel nach Anspruch 5, dadurch gekennzeichnet, dass sie die Ester der allgemeinen Formel A in einer Menge von 2 bis 30 Gew.-% enthalten.

7. Kosmetische Mittel nach einem der Ansprüche 2 bis 4, dadurch gekennzeichnet, dass der Ester unter Verwendung eines Isononylalkohols (a = 1, b = 0, c = 1, $R^1$ = H, $R^2$ = $R^3$ = $R^4$ = Methyl), eines Gemischs von Isodecylalkoholen mit Methylseitenketten, eines Gemischs von Isotridecylalkoholen mit Methylseitenketten oder von Isooctadecylalkohol ($R^1$ = $R^2$ = $R^3$ = H, $R^4$ = Methyl, a + b + c = 13) gebildet wurde.

## Revendications

1. Utilisation d'esters de formule générale A, formés à partir d'acides octanoïques à chaîne ramifiée et d'alcools à chaîne ramifiée, comme agents destinés à la répartition uniforme de principes actifs sur la peau dans des produits cosmétiques

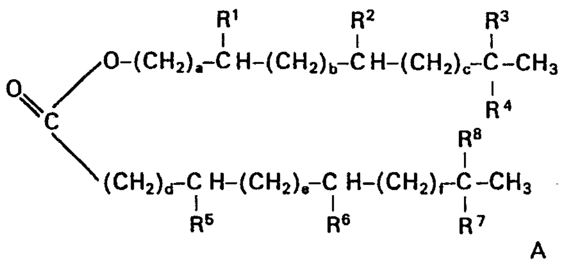

A

$R^1$, $R^2$, $R^3$, $R^4$, $R^5$, $R^6$, $R^7$, $R^8$ = H, Me, Et
a + b + c = 2–13
d + e + f = 1–2

2. Produits cosmétiques, caractérisés en ce qu'ils contiennent une quantité efficace d'esters de formule générale A, formés à partir d'acides octanoïques à chaîne ramifiée et d'alcools à chaîne ramifiée, comme agents pour la répartition uniforme de principes actifs sur la peau.

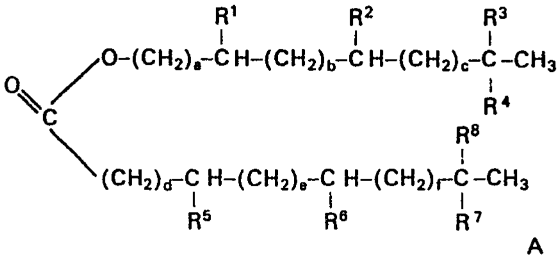

A

$R^1$, $R^2$, $R^3$, $R^4$, $R^5$, $R^6$, $R^7$, $R^8$ = H, Me, Et
a + b + c = 2–13
d + e + f = 1–2

3. Produits cosmétiques selon la revendication 2, caractérisés en ce que les esters de formule générale A sont des esters de l'acide isooctanoïque à chaîne ramifiée, qui dérivent de l'acide isooctanoïque technique, de formule générale

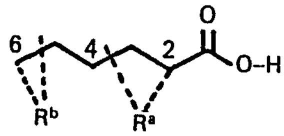

dans laquelle $R^a$ est un groupe méthyle fixé sur l'atome de carbone 2, 3 ou 4, et $R^b$ est un groupe méthyle fixé sur l'atome de carbone 5 ou 6, sous la forme d'un mélange d'isomères constitué d'acides en $C_8$ ayant une ou deux chaînes latérales méthyle, ainsi que d'alcools aliphatiques ayant de 8 à 18 atomes de carbone et 1 à 3 chaînes latérales méthyle ou éthyle.

4. Produits cosmétiques selon la revendication 2, caractérisés en ce que les esters de formule générale A sont des esters à chaîne ramifiée de l'acide 2-éthylhexanoïque, qui sont formés à partir d'acide 2-éthylhexanoïque et d'un alcool aliphatique ayant de 8 à 18 atomes de carbone et 1 à 3 chaînes latérales méthyle ou éthyle.

5. Produits cosmétiques selon la revendication 2, caractérisés en ce qu'ils contiennent des esters de formule générale A en une quantité de 1 à 90% en poids.

6. Produits cosmétiques selon la revendication 5, caractérisés en ce qu'ils contiennent les esters de formule générale A en une quantité de 2 à 30% en poids.

7. Produits cosmétiques selon l'une des revendications 2 à 4, caractérisés en ce que l'ester a été formé par utilisation d'un alcool isononylique (a = 1, b = 0, c = 1, $R^1$ = H, $R^2$ = $R^3$ = $R^4$ = méthyle), d'un mélange d'alcools isodécyliques ayant des chaînes latérales méthyle, d'un mélange d'alcools isotridécyliques ayant des chaînes latérales méthyle ou d'alcool isooctadécylique ($R^1$ = $R^2$ = $R^3$ = H, $R^4$ = méthyle, a + b + c = 13).

## Claims

1. The use of esters having the general formula A, being derived from branched-chain octanoic acids and branched-chain alcohols, as an agent for effecting a more uniform spreadability of active substances on the skin in cosmetic compositions

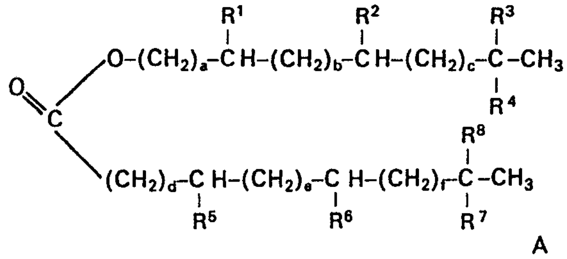

A

$R^1$, $R^2$, $R^3$, $R^4$, $R^5$, $R^6$, $R^7$, $R^8$ = H, Me, Et
a + b + c = 2–13
d + e + f = 1–2

2. Cosmetic compositions, characterized by the content of an effective amount of esters having the general formula A, being derived from branched-chain octanoic acids and branched-chain alcohols, as an agent for effecting a more uniform spreadability of active substances on the skin

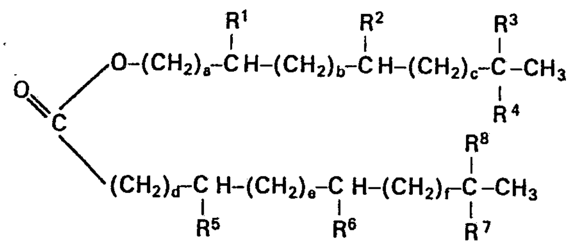

A

$R^1, R^2, R^3, R^4, R^5, R^6, R^7, R^8$ = H, Me, Et
a + b + c = 2–13
d + e + f = 1–2

3. Cosmetic compositions according to claim 2, characterized in that the esters having the general formula A are branched-chain isooctanoic acid esters which are derived from technical grade isooctanoic acid having the general formula

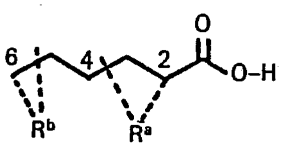

wherein $R^a$ is a methyl group on carbon atoms 2, 3 or 4 and $R^b$ is a methyl group on carbon atoms 5 or 6, being present in the form of a mixture of isomers of single or double methyl-branched $C_8$-acids and of alcohols having 8 to 18 carbon atoms as well as 1 to 3 methyl or ethyl side chains.

4. Cosmetic compositions according to claim 2, characterized in that the esters having the general formula A are branched-chain 2-ethyl-hexanoix acid esters which are derived from 2-ethyl-hexanoic acid and an aliphatic alcohol having 8 to 18 carbon atoms and 1 to 3 methyl or ethyl side chains.

5. Cosmetic compositions according to claim 2, characterized in that they comprise the esters having the general formule A in an amount of 1 to 90 percent by weight.

6. Cosmetic compositions according to claim 5, characterized in that they comprise the esters having the general formula A in an amount of 2 to 30 percent by weight.

7. Cosmetic compositions according to any of claims 2 to 4, characterized in that the ester is formed by the use of an isononyl alcohol (a = 1, b = 0, c = 1, $R^1$ = H, $R^2$ = $R^3$ = $R^4$ = methyl), of a mixture of isodecyl alcohols having methyl side chains, of a mixture of isotridecyl alcohols having methyl side chains or of isooctadecyl alcohol ($R^1$ = $R^2$ = $R^3$ = H, $R^4$ = methyl, a + b + c= 13).

1/2

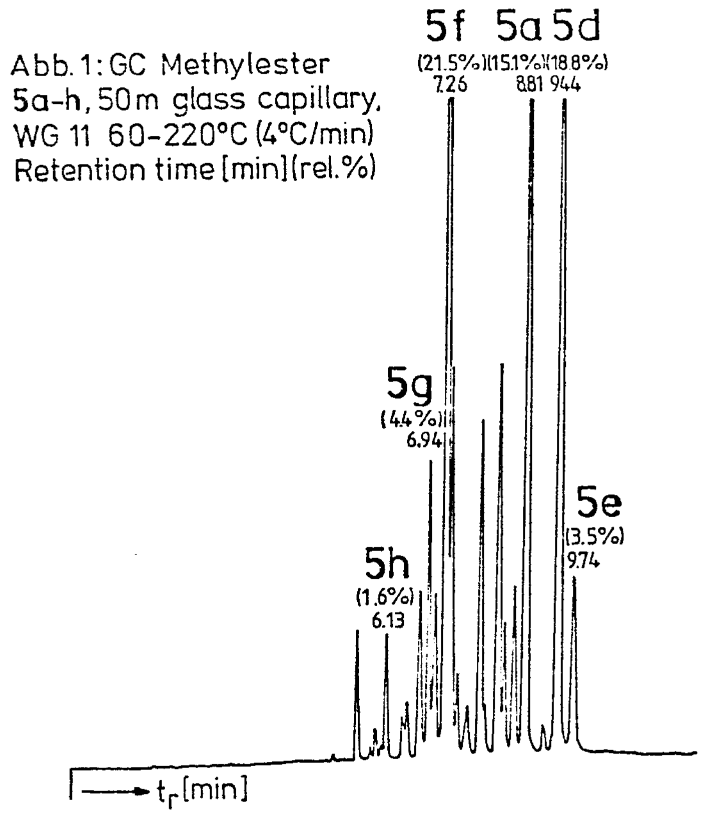

Abb. 1: GC Methylester 5a-h, 50 m glass capillary, WG 11 60-220°C (4°C/min) Retention time [min] (rel.%)

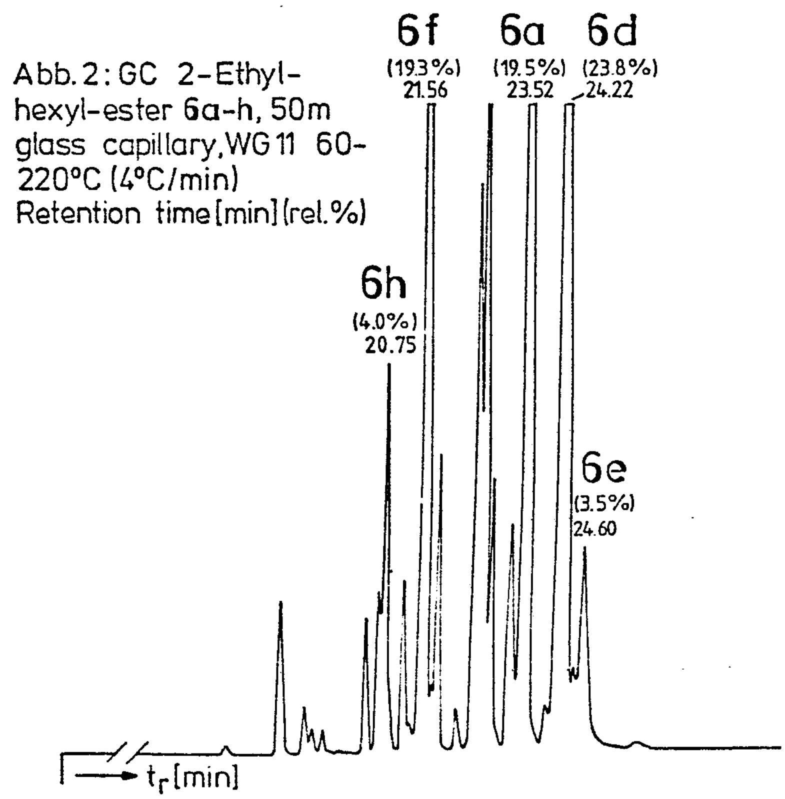

Abb. 2: GC 2-Ethyl-hexyl-ester 6a-h, 50 m glass capillary, WG 11 60-220°C (4°C/min) Retention time [min] (rel.%)

Abb.3: Massenspektren 6a,6d,6f